# EUROPEAN PATENT SPECIFICATION

(11) **EP 4 005 673 B1**
(45) Date of publication and mention of the grant of the patent: **30.07.2025**
(21) Application number: 20926157.7
(22) Date of filing: 06.10.2020
(51) Int. Cl.: B01L 3/00, C12M 1/32, C12Q 1/18, C12M 1/00, C12M 1/12

(54) **RAPID CELL CULTURE MONITORING DEVICE INCLUDING ISLAND STRUCTURE**
SCHNELLE ZELLKULTURÜBERWACHUNGSVORRICHTUNG MIT INSELSTRUKTUR
DISPOSITIF DE SURVEILLANCE DE CULTURE CELLULAIRE RAPIDE COMPRENANT UNE STRUCTURE D'ÎLOT

(30) Priority: 16.03.2020 KR 20200032125
(43) Date of publication of application: 01.06.2022
(73) Proprietor: QuantaMatrix Inc., Seoul 03082 (KR)
(72) Inventor: KIM, Jung Soo, Seoul 08505 (KR); LIM, Tae Geun, Seoul 04421 (KR); LEE, Gi Yoon, Seoul 08790 (KR); KIM, Dong Young, Seongnam-si Gyeonggi-do 13597 (KR)
(74) Representative: Prüfer & Partner mbB Patentanwälte · Rechtsanwälte
(86) International application number: PCT/KR2020/013589
(87) International publication number: WO 2021/187702

(56) References cited:
- EP-A1- 2 987 851
- EP-A1- 3 434 370
- WO-A1-2010/018499
- JP-A- 2016 061 610
- JP-A- 2017 138 112
- KR-A- 20130 089 619
- KR-B1- 101 446 526
- KR-B1- 101 711 105
- US-A1- 2013 210 131
- J. CHOI ET AL: "A rapid antimicrobial susceptibility test based on single-cell morphological analysis", SCIENCE TRANSLATIONAL MEDICINE, vol. 6, no. 267, 17 December 2014 (2014-12-17), pages 267ra174 - 267ra174, XP055618055, ISSN: 1946-6234, DOI: 10.1126/scitranslmed.3009650
- SHIN-YI CINDY CHEN ET AL: "Microfluidic array for three-dimensional perfusion culture of human mammary epithelial cells", BIOMEDICAL MICRODEVICES, KLUWER ACADEMIC PUBLISHERS, BO, vol. 13, no. 4, 10 May 2011 (2011-05-10), pages 753 - 758, XP019921901, ISSN: 1572-8781, DOI: 10.1007/S10544-011-9545-3

## Description

### Technical Field

The present disclosure relates to a cell culture test device, and more specifically to a cell culture test device including island structures and designed such that dispensed fluids are evenly distributed in wells of a chip, facilitating accurate observation.

### Background Art

Timely prescription of antibiotics for human patients infected with infectious agents greatly affects the survival of the patients. For correct antibiotic prescription, it is first necessary to determine the concentrations of specific antibiotics at which the growth of infection pathogenic strains is inhibited. This determination process is called antibiotic susceptibility testing.

According to a typical antibiotic susceptibility test, whether infectious pathogens are grown after culture in microwells containing various concentrations of a mixture of a culture medium and an antibiotic for about 18 hours is determined by transmittance measurements. A number of products are currently being developed to minimize the time required for culturing infectious pathogens and automate the testing process.

First, miniaturization of culture environments for infectious pathogens is a basic requirement for minimizing the culture time of the infectious pathogens. For such miniaturization and automation, there is a rapidly growing tendency to introduce disposable plastic plates having microscale structures into which microfluids are dispensed and which can be adjusted to desired shapes and sequences.

The present applicant has succeeded in developing an antibiotic susceptibility test system in which a microfluidic plate is introduced such that the growth of bacteria is determined by microscopic imaging. The use of this system is advantageous in that antibiotic susceptibility testing can be completed within a few hours but has limitations in that a complicated shape of channels is required for three-dimensional cell immobilization and culture medium and drug supply and a culture medium and a drug are difficult to spread over long distances.

### Prior art documents:

D1 : J. CHOI et al., "A rapid antimicrobial susceptibility test based a single- cell morphological analysis", Science Translational Medicine, vol. 6, no. 267, December 17, 2014, pages 267ra174-267ra174, XP055618055, ISSN: 1946-6234, DOI: 10. 1126/scitranslmed.3009650
D2 : EP 2 987 851 A1 (QUANTA MATRIX CO LTD; UNI. SEOUL NAT R & DB FOUND [KR]) February 24, 2016
D3 : EP 3 434 370 A1 (QUANTA MATRIX CO LTD [KR]) January 30, 2019
D4 : WO 2010/018499 A1 (KONINKL PHILIPS ELECTRONICS NV [NL]; CATTANEO STEFANO [NL] et al.) February 18, 2010
D5 : SHIN-YI CINDY CHEN et al., BIOMEDICAL MICRODEVICES, KLUWER ACADEMIC PUBLISHERS, BO, vol. 13, no. 4, May 10, 2011, pages 753-758, XP019921901, ISSN: 1572-8781, DOI: 10.1007/S10544-011-9545-3
D6 : US 2013/21 01 31 A1 (RENKEN CHRISTIAN W [US]), August 15, 2013

D1 is a NPL (journal paper) related to a rapid antibiotic susceptibility test, which is single-cell morphological analysis (SCMA) that can determine antimicrobial susceptibility by automatically analyzing and categorizing morpho-logical changes in single bacterial cells under various antimicrobial conditions.

D2 is a patent document with respect to a microfluidic multi-well-based cell culture testing device, which has an array structure of a plurality of aligned microfluidic well units and each of the microfluidic well units comprises an inlet through which a first fluid enters, an accommodation compartment adapted to accommodate a second fluid therein, a microfluidic channel through which the first fluid flows, and an air outlet adapted to facilitate the entering of the first fluid.

D3 is a patent document and discloses a multi-well-based cell culture test device having an array structure of a plurality of aligned well units. Each of the well units includes a first sub-well adapted to accommodate a first fluid, a second sub-well adapted to accommodate a second fluid, and a barrier located between the first sub-well and the second sub-well to partition the first sub-well and the second sub-well.

D4 is a patent document disclosing a method and apparatus for performing cellular susceptibility testing via a concentration gradient in a microfluidic device including a microfluidic channel, comprising the steps of trapping one or more single cells to be tested in one or more defined positions on an inner surface of the channel; applying a stable and predictable concentration gradient of a substance through the channel with the cells to be tested; incubating the cells to be tested within the concentration gradient.

D5 is a NPL (journal paper) disclosing a microfluidic perfusion array on a 96-well plate format capable of long term three dimensional extracellular matrix (3D ECM) culture within biomimetic microchambers, in which perfusion is generated using gravity and surface tension forces, allowing the array to be operated without any external pumps.

D6 is a patent document and discloses a filter device including a well, at least one top access opening to the well, and a horizontally-disposed filter, which includes an inner surface at least partially defining the well, also includes a first well chamber to accommodate a fluid and a second well chamber to accommodate a fluid, both of which are separate chambers, and the inner surface at least partially defines the first well chamber and at least partially defines the second well chamber.

### Detailed Description of the Invention

### Problems to be Solved by the Invention

The present disclosure has been made in an effort to overcome the above-described limitations and intends to provide a cell culture test device including island structures and designed such that dispensed fluids are evenly distributed in wells of a chip, facilitating accurate observation according to claim 1.

### Means for Solving the Problems

The present invention provides a cell culture test device according to claim 1 and a cell analysis method according to claim 13. Preferred embodiments are set forth in the sub-claims to claims 1 and 13, respectively.
One aspect the present disclosure provides a cell culture test device including a plurality of well units, each of which includes a first inlet portion through which a first fluid is introduced, a first accommodation space communicating with the first inlet portion to accommodate a flow of the first fluid introduced through the first inlet portion, a second inlet portion through which a second fluid is introduced, a second accommodation space in which the introduced second fluid is accommodated wherein the second accommodation space connects to the inner surface of the first accommodation space to form an interface perpendicular to a bottom surface between the first fluid and the second fluid,
and a third inlet portion through which an antibiotic had been loaded, which third inlet portion has been formed above and has been connected with an island structure extending from the wall surface of the second accommodation space, wherein the antibiotic loaded from the third inlet portion is fixedly accommodated on the inner and outer surfaces of the island structure.

In one embodiment, the center of the island structure may coincide with the center of the first accommodation space and the island structure may be spaced a distance from the first accommodation space.

In one embodiment, the lower end of the island structure may be spaced 0.1 to 1 mm from the bottom surface of the second accommodation space and the island structure may have an open bottom surface.

In one embodiment, the introduced second fluid may be in contact with the inner and outer surfaces of the island structure, dissolve the antibiotic fixed to the inner and outer surfaces of the island structure, and diffuse the dissolved antibiotic into the first fluid.

In one embodiment, the thickness and width of the first accommodation space may be determined such that the first accommodation space is filled with the first fluid introduced through the first inlet portion by capillary action.

In one embodiment, the first accommodation space may have a width of 0.3 to 2 mm and a thickness of 100 to 500 µm.

In one embodiment, the side surface of the second accommodation space may be in communication with the inner surface of the first accommodation space such that the first fluid and the second fluid meet each other and mass transfer to the first fluid occurs.

In one embodiment, the first accommodation space may be an open microfluidic structure whose at least one surface is open and the open surface of the first accommodation space may be connected to the side surface of the second accommodation space through a capillary valve.

In one embodiment, the thickness and width of the capillary valve structure may be determined such that the introduced first fluid does not enter the second accommodation space during or after flow into the first accommodation space.

In one embodiment, the capillary valve may have a thickness of 100 to 500 µm and a width of 100 µm to 2 mm.

In one embodiment, the cell culture test device may include bonding means disposed on the rear surface thereof to prevent leakage of the first fluid and the second fluid.

In one embodiment, the bonding means may be an optically transparent film.

A further aspect of the present disclosure provides a cell analysis method using a cell culture test device having an array structure of a plurality of aligned well units, each of which includes a first inlet portion through which a mixture solution of a gelling agent-containing liquid medium and a biological agent as a first fluid is introduced, a second inlet portion through which a solvent selected from water, cell culture media, dimethylformamide (DMF), and dimethyl sulfoxide (DMSO) as a second fluid is introduced, a first accommodation space communicating with the first inlet portion in which the introduced first fluid is accommodated, a second accommodation space in which the introduced second fluid is accommodated, an island structure accommodated in the second accommodation space, and a third inlet portion through which an antibiotic is loaded into the island structure has been formed above the island structure and has been connected therewith through an island-connecting structure extending from the inner wall surface of the second accommodation space, the method including (a) loading the antibiotic into the third inlet portion formed above the island structure to fix the antibiotic to the inner and outer surfaces of the island structure, (b) introducing the first fluid into the first inlet portion, allowing the introduced first fluid to flow into the first accommodation space, and gelling the mixture solution to form a solid thin film, (c) introducing the second fluid into the second inlet portion, allowing the second fluid to be accommodated in the second accommodation space and come into contact with the island structure to disperse or dissolve the antibiotic, and allowing the second fluid to come into contact with the solid thin film of the first fluid in a connection portion between the first accommodation space and the second accommodation space to diffuse the antibiotic into the solid thin film at their interface formed perpendicular to a bottom surface between the first fluid and the second fluid, and (d) observing changes of the biological agent on a single cell basis at the interface between the solid thin film of the first fluid and the second fluid and in the solid thin film of the first fluid.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

### Effects of the Invention

The rapid cell culture test device of the present disclosure includes island structures and is designed for uniform distribution and rapid diffusion of an antibiotic, allowing for accurate testing compared to conventional antibiotic test devices. The rapid cell culture test device of the present disclosure uses cells immobilized in solid thin films to observe the effects of the antibiotic, enabling rapid antibiotic susceptibility testing compared to conventional devices.

In addition, the rapid cell culture test device of the present disclosure can generally observe changes in the growth of single bacteria within several tens of minutes (usually 30 minutes) and changes in the growth of single colonies of bacteria within 4 to 6 hours. Accordingly, the rapid cell culture test device of the present disclosure can more accurately and rapidly determine the effects of a bioactive agent on a biological agent than conventional devices.

Furthermore, the rapid cell culture test device of the present disclosure can observe changes of a biological agent responding to a bioactive agent on a single cell colony basis to determine the minimum inhibitory concentration (MIC) or minimum biofilm eradication concentration (MBEC) of the bioactive agent, thus being very useful for biofilm assay as well as antibiotic susceptibility testing.

### Brief Description of the Drawings

FIG. 1 illustrates one embodiment of a cell culture test device.
FIG. 2 illustrates a well unit of a cell culture test device according to one embodiment of the present disclosure.
FIG. 3 illustrates an antibiotic susceptibility test using a first fluid and a second fluid introduced into a well unit.
FIG. 4 illustrates a first fluid introduced into a microfluidic channel of a well unit: (a) Plan view and (b) cross-sectional view taken along line A-A' of (a).
FIG. 5 schematically illustrates the behaviors of a fluid supplied through a second inlet portion and a dried antibiotic attached to a third inlet portion.
FIG. 6 illustrates plan, perspective, and cross-sectional views of various shapes of island structures and island-connecting structures 143 according to exemplary embodiments of the present disclosure.
FIG. 7 illustrates a mold used to manufacture a cell culture test device according to one embodiment of the present disclosure.
FIG. 8 illustrates rear bonding means and an adhesive applied to the rear bonding means according to exemplary embodiments of the present disclosure.
FIG. 9 shows a high magnification image of a solid thin film according to one embodiment of the present disclosure.
FIG. 10 shows observation results of solid thin films when colistin was used at various concentrations ((a) 0 mg/ml, (b) 0.12 mg/ml, (c) 0.25 mg/ml, (d) 0.5 mg/ml, (e) 1 mg/ml, and (f) 2 mg/ml) according to exemplary embodiments of the present disclosure.
FIG. 11 shows images illustrating the behaviors of a fluid when island structures were spaced various distances according to exemplary embodiments of the present disclosure.

### Mode for Carrying out the Invention

Embodiments of the present disclosure will now be described in detail with reference to the accompanying drawings.

However, the present disclosure may be embodied in many different forms and should not be construed as limited to the embodiments set forth herein. Rather, these embodiments are provided so that this disclosure will be thorough and complete, and will fully convey the scope of the present disclosure to those skilled in the art. In the drawings, the dimensions, such as widths and thicknesses, of elements may be exaggerated for clarity. The drawings are explained from an observer's point of view. It will be understood that when an element is referred to as being "on" another element, it can be directly on the other element, or one or more intervening elements may also be present therebetween. Those skilled in the art will appreciate that many modifications can be made without departing from the scope of the disclosure. Throughout the accompanying drawings, the same reference numerals are used to designate substantially the same elements.

On the other hand, terms used herein are to be understood as described below. While such terms as "first" and "second," etc., may be used to describe various elements, such elements must not be limited to the above terms. The above terms are used only to distinguish one element from another. For example, a first element may be referred to as a second element, and likewise a second element may be referred to as a first element.

As used herein, the singular forms "a", "an" and "the" are intended to include the plural forms as well, unless the context clearly indicates otherwise. It will be further understood that the terms "include(s)", "including", "have (has)" and/or "having", when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Respective steps of the methods described herein may be performed in a different order than that which is explicitly described. In other words, the respective steps may be performed in the same order as described, simultaneously, or in a reverse order.

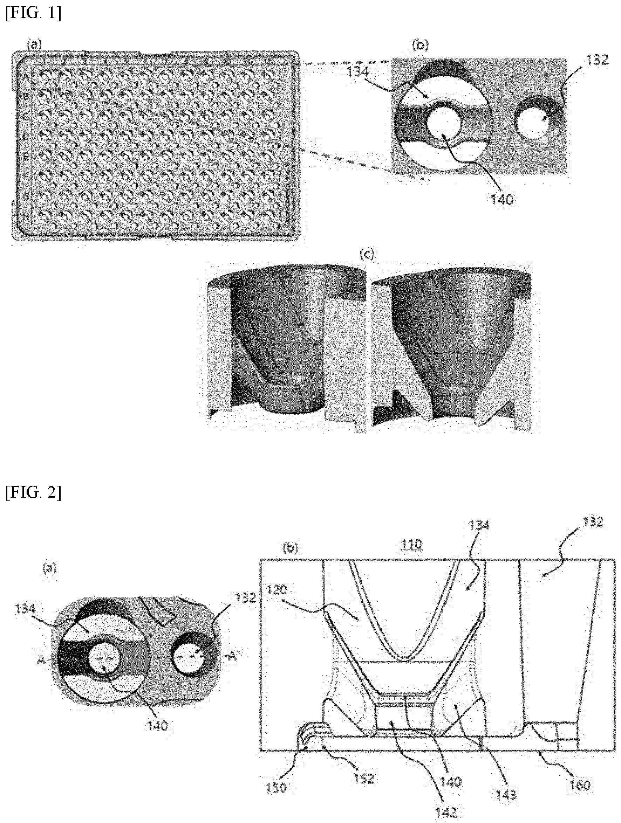

According to one aspect of the present disclosure, there is provided a cell culture test device including a plurality of well units as defined in claim 1. FIG. 1 illustrates one embodiment of the cell culture test device. In FIG. 1, (a) illustrates the constitution of the cell culture test device, (b) illustrates a partially enlarged view of the cell culture test device, and (c) illustrates a cross-sectional view of one of the well units. Referring to FIG. 1, the plurality of well units 110 are aligned in the cell culture test device. The plurality of the aligned well units 110 may have overall dimensions similar to the wells of a commercial multi-well plate. The centers of the well units 110 may coincide with the centers of the wells of a commercial multi-well plate. Each of the well units 110 includes a first inlet portion 132 through which a first fluid is introduced, a second inlet portion 134 through which a second fluid is introduced, a first accommodation space 150 communicating with the first inlet portion to accommodate a flow of the first fluid introduced through the first inlet portion 132, and a second accommodation space 120 in which the second fluid is accommodated.

Multi-well plates are standard tools for processing and analyzing a large number of samples in chemical, biochemical, and/or biological assays. Multi-well plates may take various forms, sizes, and shapes. Generally, multi-well plates are designed to have standard sizes and shapes and have standard arrangements of wells. For compatibility with such multi-well plates, the plurality of well units may have standard arrangements of wells, including those found in 96-well plates (12 × 8 array of wells), 384-well plates (24 × 16 array of wells), and 1536-well plates (48 × 32 array of wells). The arrangements of the plurality of well units may be identical or similar to those of various commercially available multi-well plates. The cell culture test device 100 is readily compatible with various conventional biological analysis techniques because its dimensions are similar to those of a commercial multi-well plate. Accordingly, the well units may be arranged in a 1 × 1, 1 × 2, 1 × 4, 2 × 4, 4 × 6, 12 × 8, 24 × 16 or 48 × 32 matrix.

FIG. 2 illustrates (a) a well unit of a chip for antibiotic testing and (b) a cross-sectional view taken along line A-A' of (a).

### [Locations of the first inlet portions and the second inlet portions]

The first inlet portion 132 and the second inlet portion 134 are usually located in the upper portion of each of the well units 110. Each of the first inlet portion 132 and the second inlet portion 134 may have a fully or partially open shape. One or more openings may be formed in each of the first inlet portion 132 and the second inlet portion 134. The first fluid and the second fluid may be introduced into the well unit 110 through the first inlet portion 132 and the second inlet portion 134, respectively, by pipetting or injection.

### [Features of the first inlet portions]

The first fluid is introduced through the first inlet portion 132. The lower end of the first inlet portion 132 communicates with the first accommodation space 150 so that the introduced first fluid can be supplied to the first accommodation space 150. Since the first inlet portion 132 is simply used for supply of the first fluid, the first inlet portion 132 is preferably designed in a cylindrical shape, a conical shape with an open top end or a tapered shape for a smooth flow of the supplied first fluid. Particularly, when designed in a tapered shape, the first inlet portion 132 is easy to release from a mold after injection molding. In addition, the inlet with an enlarged diameter ensures easy introduction of the first fluid. Furthermore, the introduced first fluid is collected at the point where the first inlet portion 132 and the first accommodation space 150 meet each other and supplied to the first accommodation space 150. When a smooth flow of the first fluid into the first accommodation space is ensured, the first inlet portion may be in communication with the second inlet portion or may be accommodated in the second inlet portion.

### [Features of the second inlet portions]

The second fluid is introduced into the second accommodation space 120 through the second inlet portion 134. The second inlet portion 134 may be located on top of the second accommodation space 120. The second inlet portion 134 provides a space for introduction of the second fluid and the second accommodation space 120 accommodates a third inlet portion 140 and an island structure 142 connected to each other through an island-connecting structure 143. Thus, it is preferable that the second inlet portion 134 is larger in diameter than the first inlet portion 132. When the second fluid is controlled so as not to enter the first inlet portion, the second inlet portion may also accommodate the first inlet portion. The second inlet portion 134 may have the same cross-sectional shape as the second accommodation space 120. Alternatively, the shape of the second inlet portion 134 may be partially deformed to specify the introduction location and improve the convenience of introduction. The deformed portion may aid in guiding smooth entry of injection means *(e.g.,* a pipette) for introduction of the second fluid. Thus, an accurate amount of the second fluid can be introduced into an accurate location.

### [Features of the first fluid and the second fluid]

Each of the first and second fluids typically includes 70% to 99% by weight, preferably 85% to 99% by weight, most preferably 93% to 99% by weight of water as a dispersion medium or solvent. For example, the first fluid may be a mixture solution of a gelling agent-containing liquid medium and a biological agent. When the content of water in the first fluid is within the range defined above, the water can exhibit optimal performance as a dispersion medium.

### [Features of the liquid medium in the first fluid]

The liquid medium may include at least about 95% of water and may be gelled in the presence of a gelling agent. For example, the gelling agent may be agar, agarose, gelatin, alginate, collagen or fibrin. The use of agar or agarose is preferred. For example, agar may be used in an amount of 0.3 to 4% by weight in the liquid medium. The liquid medium usually requires no nutrients. In some examples, however, the liquid medium may include nutrients. The liquid medium may be a solution including a medium stimulating the division of particular cells. If the agar content is less than 0.3% by weight, the liquid medium may not be gelled. Meanwhile, if the agar content exceeds 4% by weight, the liquid medium may be excessively gelled, making it difficult to diffuse the biological agent.

### [Features of the biological agent in the first fluid]

Examples of suitable biological agents include viruses, bacteria, fungi, algae, protozoa, parasitic pathogens, human and mammalian cells, and biofilms. The biological agent may be a mixture of an infectious biological agent (*e.g.,* a bacterial, viral or fungal strain) and blood cells. For example, the biological agent may be blood collected from a human infected with bacteria. In this case, a medium stimulating the division of only the bacterial cells can be used to distinguish the bacterial cells from the blood cells based on their different sizes. The biological agent may grow in a liquid or solid medium and the growth of the biological agent may be affected by the kind and concentration of a foreign bioactive agent. The density of the biological agent in the mixture solution is from 10² to 10¹⁰ cells/ml, preferably from 10⁴ to 10¹⁰ cells/ml, more preferably from 10⁵ to 10⁹ cells/ml. If the density of the biological agent is below the lower limit, it may be difficult to perceive the location of the biological agent. Meanwhile, if the density of the biological agent exceeds the upper limit, it may be difficult to perceive the individual state of the biological agent.

### [Features of the first accommodation spaces]

The first accommodation space 150 communicates with the first inlet portion 132 so that the first fluid introduced from the first inlet portion 132 can fill the first accommodation space 150. In general, the first accommodation space 150 may have an open microfluidic channel structure. For example, the first accommodation space 150 may be in the level of hundreds of µm to several mm in width and hundreds of µm in depth (or thickness). These dimensions facilitate subsequent imaging and enable filling of the first fluid in the first accommodation space 150 by capillary action. The first fluid may be a liquid medium containing a gelling agent. In this case, the first fluid is gelled after the lapse of a predetermined time, resulting in the formation of a solid thin film filling the microfluidic channel. The first accommodation space may be in the shape of a doughnut surrounding the second accommodation space. The first fluid introduced into the first accommodation space comes into contact with the second fluid simultaneously with gelling to form a solid thin film. The first accommodation space may be in the shape of a doughnut surrounding the outer surface of the second accommodation space to maximize the contact area between the solid thin film and the second fluid. Alternatively, when the second accommodation space has a polygonal shape such as a quadrangular or triangle shape, the first accommodation space is preferably adapted to the shape of the second accommodation space such that its contact portion with the second fluid located along the inner circumference of the first accommodation space is maximized.

The introduced first fluid may be gelled to form a solid thin film in the first accommodation space 150. The first fluid introduced through the first inlet portion undergoes gelling. The thickness of the solid thin film may be determined depending on the depth of the first accommodation space 150. The thickness of the first accommodation space may be in the range of 1 µm to 500 µm, 1 µm to 100 µm or 100 µm to 500 µm. The depth of the first accommodation space is preferably 500 µm or less, more preferably 100 µm to 500 µm. If the thickness of the first fluid accommodation space exceeds the upper limit defined above, the thickness of the thin film becomes large, which increases the required amount of a sample or makes accurate observation difficult. Meanwhile, if the thickness of the first fluid accommodation space less than the lower limit defined above, the amount of observable biological agent per same area is too small, it makes to observe and determination difficult.

In consideration of the size of an image area, the width of the first accommodation space may be 100 µm to 5 mm, 300 µm to 5 mm, 500 µm to 3 mm or 1 mm to 3 mm. The width of the microfluidic channel is preferably 0.3 mm to 2 mm. If the width of the first fluid accommodation space is less than the lower limit defined above, a narrow observable field of view is secured, making accurate observation difficult. Meanwhile, if the width of the first fluid accommodation space exceeds the upper limit defined above, the required amount of a sample increases, which is inefficient.

### [Dimensions of the solid thin film in the first accommodation space]

The thickness and width of the solid thin film are determined depending on the depth and width of the microfluidic channel. As used herein, the term "thin film" refers to a thin layer that has a thickness sufficient to immobilize the biological agent and to observe single cells. The thickness of the thin film is typically in the range of 1 µm to 5 mm, 1 µm to 3 mm, 1 µm to 2 mm, 1 µm to 1.5 mm, 1 µm to 1 mm, 1 µm to 800 µm, 1 µm to 500 µm, 1 µm to 100 µm, 10 µm to 3 mm, 100 µm to 500 µm, 10 µm to 1 mm, 100 µm to 1 mm, 200 µm to 1 mm or 500 µm to 1 mm, but is not particularly limited to this range. The thickness of the solid thin film may correspond to the size of a side of the solid thin film in a direction perpendicular to a side of the solid thin film to be observed. Within the thickness range of the solid thin film defined above, the biological agent immobilized in the solid thin film can be observed on a single cell basis.

### [Interfacial properties of the solid thin film in the first accommodation space]

The first accommodation space 150 communicates with the side surface of the second accommodation space 120 such that the first fluid and the second fluid come into contact with each other to form an interface perpendicular to the bottom surface. In a prior art cell culture test device, the first fluid and the second fluid form an interface parallel to the bottom surface. In this case, however, changes caused by an antibiotic can be observed only at a specific height. Particularly, when an appropriate biological agent (*e.g.,* a bacterial or fungal species) is not located at the specific height for observation, no changes may be observed or the test reliability may deteriorate. Further, a height variation of the horizontal interface may be caused by a volume variation of the first fluid and a concentration gradient of the antibiotic may occur. In this case, the concentration-dependent effect of the antibiotic may further deteriorate the reliability of the test results. In contrast, according to the present disclosure, the connection of the second accommodation space to the inner surface of the first accommodation space leads to the formation of an interface perpendicular to the bottom surface. The location of the interface can be accurately found upon observation. Particularly, observation of the interface and its surroundings facilitates observation of changes of the biological agent present in a smaller amount in the first fluid.

When the inner surface of the first accommodation space is connected to the second accommodation space such that the center of the second accommodation space coincides with the center of the first accommodation space, the center of the island structure also coincides with the centers of the first and second accommodation spaces, and as a result, the interface between the first fluid and the second fluid is formed at the same distance from the center of the island structure. Particularly, when an antibiotic introduced into the third inlet portion or attached to the island structure is diffused into the second fluid and transferred to the interface, the concentration of the antibiotic can be made uniform because the interface is at the same distance from the initial position of the antibiotic. To this end, the inner circumferential surface of the first accommodation space may be designed to form the side surface of the second accommodation space 120. That is, the entire inner circumferential surface of the first accommodation space is preferably in communication with the lower side surface of the second accommodation space. The first accommodation space is preferably connected to the lower side surface of the second accommodation space through a capillary valve to prevent the first fluid introduced through the outer circumferential surface of the first accommodation space from passing through the inner circumferential surface of the first accommodation space in communication with the lower side surface of the second accommodation space.

### [Features of the second accommodation spaces]

The second fluid introduced through the second inlet portion 134 is accommodated in the second accommodation space 120. The second accommodation space 120 accommodates the island structure 142 and the third inlet portion 140 connected to each other through the island-connecting structure 143. The second accommodation space 120 may be in the form of a well that has a sufficient space to accommodate the second fluid. The volume of the second accommodation space is not particularly limited and may be large enough to observe responses for a long time after introduction of the second fluid. The volume of the second accommodation space is preferably 100 µl to 2000 µl. If the volume of the second accommodation space is less than 100 µl, the reduced size of the well is not suitable for observation. Meanwhile, if the volume of the second accommodation space exceeds 2000 µl, large amounts of a sample and reagents are required, resulting in a reduction in efficiency.

### [Features of the island-connecting structures]

The third inlet portion 140 and the island structure 142 are placed in the second accommodation space 120 by the island-connecting structure 143. The second fluid is preferably introduced into a space between the island-connecting structure 143 and the inner wall of the second accommodation space 120. To this end, the island-connecting structure 143 extends inward from the wall surface of the second accommodation space 120. The third inlet portion 140 and the island structure 142 may be located in the central portion of the island-connecting structure 143. The island structure should be positioned at a distance below a predetermined level from the second accommodation space. Thus, the island-connecting structure 143 preferably extends from the inner wall surface of the second accommodation space 120 toward the center of the lower end of the second accommodation space 120.

The third inlet portion 140 and the island structure 142 may be accommodated in the second accommodation space 120 by one island-connecting structure 143. Alternatively, the island-connecting structure 143 may be provided in plurality. The third inlet portion 140 and the island structure 142 are preferably connected to each other through two or more island-connecting structures 143, more preferably two to four island-connecting structures 143. Two extending island-connecting structures are most preferred for easy introduction of the second fluid.

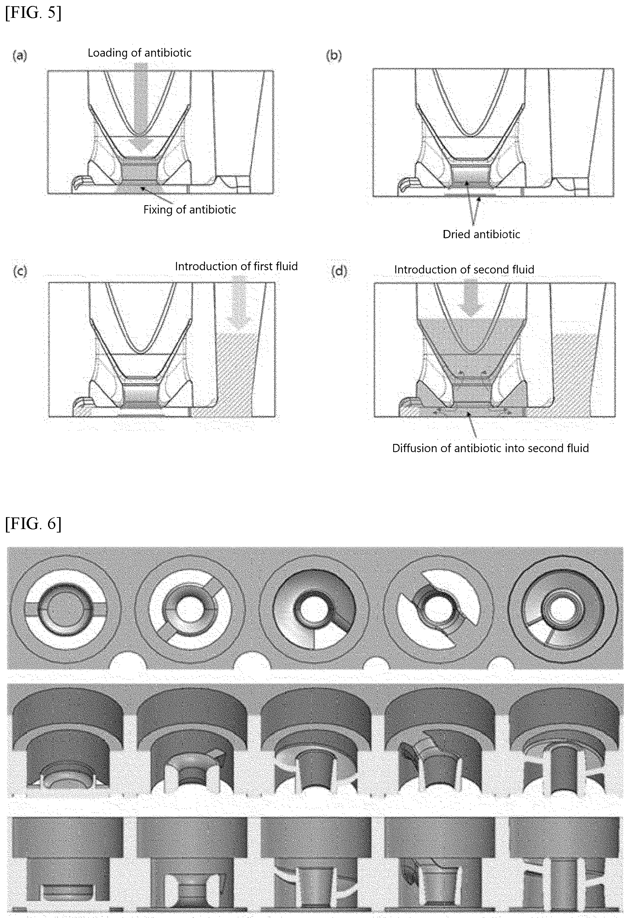

The island-connecting structure 143 may vary in shape. FIG. 6 illustrates plan, perspective, and cross-sectional views of various shapes of island structures and island-connecting structures 143. As illustrated in FIG. 6, the island structure may have an open channel (2, 3, 4 or 5 in the top row of FIG. 6) or closed basket shape (1 in the top row of FIG. 6). The ability of the island structure to trap the antibiotic is more important than the shape of the island structure. The open channel shape is more advantageous for antibiotic diffusion during introduction of the second fluid than the closed basket shape. However, an optimized design is required to prevent the antibiotic from leakage into the open space from when the antibiotic is introduced until when the antibiotic is dried.

The island-connecting structure 143 connects between the island structure and the second accommodation space. One island-connecting structure 143 may be used to fix the island structure. Two or more symmetric or wide island-connecting structures 143 are recommended for their stable release from a mold after injection molding. Two or more symmetric or singly threaded island-connecting structures 143 are preferable to avoid interference with dispensing of the second fluid. In the case where a plurality of island-connecting structures 143 are provided, the number, shape, and size of the island-connecting structures 143 need to be optimized to ensure stable release from a mold after injection molding and secure a space for smooth dispensing of the second fluid. Threaded island-connecting structures 143 ensure the highest stability during release due to their large contact area. However, threaded island-connecting structures 143 are difficult to use because the second fluid is introduced only in one direction. It is preferable to use two island-connecting structures 143 that have the same thickness as the diameter of the island structure and are symmetrically connected to the second accommodation space.

### [Features of the third inlet portions]

The antibiotic is loaded through the third inlet portion 140. The third inlet portion 140 may be connected to the upper portion of the island structure and accommodated in the second accommodation space 120. The third inlet portion 140 is in communicate with the underlying island structure where the loaded antibiotic can be accommodated (see FIG. 5). Since the third inlet portion 140 is simply used for supply of the antibiotic, the third inlet portion 140 is preferably designed in a cylindrical shape, a conical shape with an open top end or a tapered shape for a smooth flow of the supplied antibiotic into the island structure.

### [Features of the island structures]

The island structure 142 is fixed inside the second accommodation space by the island-connecting structure 143. The antibiotic loaded through the third inlet portion is accommodated in the island structure 142 or the dried antibiotic (in a solid form) is attached to the island structure 142. The loaded liquid antibiotic may be accommodated in the island structure 142. Alternatively, the loaded liquid antibiotic may escape through a bottom opening of the island structure and may be accommodated in a space between the island structure and the bottom of the second accommodation space 120. As long as the liquid antibiotic does not leak out of the island structure 142 due to surface tension, the antibiotic loaded into the island structure 142 may be placed away from the bottom of the second accommodation space 120. The antibiotic may be dried and attached to the island structure 142 or between the island structure 142 with an open bottom and the bottom surface of the second accommodation space 120. The antibiotic may be dispersed or dissolved in the introduced second fluid. In the case where the antibiotic is loaded into the island structure with an open bottom and placed away from the bottom of the second accommodation space, a larger volume of the antibiotic is accommodated and the diffusion path of the dried antibiotic is shortened after introduction of the second fluid, which is advantageous for smooth diffusion of the antibiotic. The shape of the antibiotic should be maintained by surface tension in the space between the island structure and the second accommodation space to confine the loaded liquid antibiotic to the space between the island structure and the second accommodation space and prevent the loaded liquid antibiotic from spreading out of the island structure. At this time, the island structure is preferably spaced 0.1 to 1 mm from the second accommodation space to maintain the surface tension of the antibiotic. If the distance is less than 0.1 mm, the space required for diffusion is reduced, and as a result, a lot of time is taken to test the susceptibility to the antibiotic. Meanwhile, if the distance exceeds 1 mm, the shape of the antibiotic cannot be maintained due to surface tension, causing leakage of the antibiotic out of the island structure.

### [Features of the antibiotic in the island structures]

The third inlet portion 140 may include the antibiotic in a solid form. In this case, the antibiotic may be in a dry state (see (b) of FIG. 5). The dried antibiotic may be dissolved in a solvent selected from water, cell culture media, dimethylformamide (DMF), and dimethyl sulfoxide (DMSO). The solvent may be introduced by pipetting such that the antibiotic in a solid form is completely dissolved and the resulting solution has a uniform concentration ((d) of FIG. 5). Any drying process that does not deteriorate the efficacy of the antibiotic may be used without limitation to dry the antibiotic. The antibiotic is preferably dried by room temperature drying, heat drying, freeze drying or vacuum drying, most preferably heat drying. The antibiotic thus dried is introduced into the third inlet portion. Alternatively, the antibiotic solution may be introduced into the third inlet portion and dried by the drying process mentioned above.

An antibiotic-containing fluid may be directly introduced into the third inlet portion 140. In this case, the antibiotic-containing fluid may be a solution of the antibiotic in a solvent. The solvent may be the same as the second fluid. Alternatively, the antibiotic may be dissolved in a solvent different from the second fluid for ease of storage and handling. In this case, the solvent is preferably selected from solvents that are easily mixed or diffused with the second fluid. It is preferable that the antibiotic has a constant surface tension such that it is dried while maintaining its shape in the space between the island structure and the second accommodation space. Here, the distance between the island structure and the second accommodation space is determined based on water whose viscosity is similar to those of most antibiotics.

### [Features of the capillary valves]

The inner surface of the second accommodation space is connected to the inner surface of the first accommodation space through the capillary valve 152. The capillary valve 152 prevents the first fluid from entering the second accommodation space 120 during or after flow into the first accommodation space 120. The thickness and angle of the capillary valve 152 may be determined such that the flow of the first fluid is controlled. The first fluid entering the first accommodating portion having a small width has a high surface tension due to its interface with air. The interface of the first fluid in the first accommodation space forms a contact angle and a surface tension in a direction supporting the flow direction. In contrast, the interface of the first fluid forms a contact angle and a surface tension in a direction resisting its entry into the second accommodation space due to the varying edge angle at the boundary between the second accommodation space and the first accommodation space. The capillary valve can be used to release air from the channel and eliminate a pressure difference between the first inlet portion and the first accommodation space when the first fluid is supplied to the first accommodation space, enabling uniform filling of the first accommodation space 150 with the first fluid. For easy mass diffusion of the second fluid, the thickness and width of the capillary valve are preferably determined such that the diffusion path is not disturbed. The thickness and width of the capillary valve are typically 100 to 500 µm and 100 µm to 2 mm, respectively. However, the width of the capillary valve may be freely selected when the thickness of the capillary valve is the same as that of the first accommodation space. Within the thickness and width ranges defined above, air can be easily released from the first accommodation space 150 and the second fluid can be easily diffused without leakage of the first fluid. If the thickness and width of the capillary valve are less than the respective ranges, air is not easily released and easy mass diffusion of the second fluid is not attained. Meanwhile, if the thickness and width of the capillary valve exceed the respective ranges, the first fluid leaks to the second accommodation space, which increases the required amount of a sample or makes it difficult to accurately detect test results.

### [Features related to chip manufacturing]

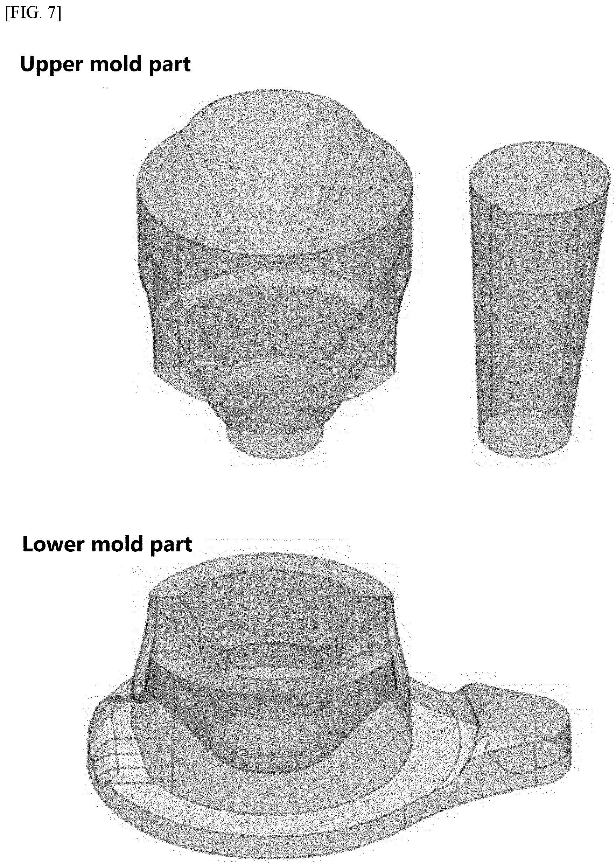

A conventional disposable plastic chip (or a multi-well plate) is typically manufactured by injection molding and includes wells designed so as to have a shape in which the upper end is wider than the lower end. Due to this downwardly tapered shape, the chip is easy to remove from a mold after injection molding and undergoes minimal damage during removal from the mold. However, in the case where the downwardly tapered product includes island structures accommodated in second accommodation portions and first accommodation spaces whose diameter is larger than that of second accommodation spaces at the bottom thereof, as in the present disclosure, a mold having an undercut shape is required because of the characteristics of the product. Due to the shape of the mold, the product cannot be injection molded at one time. In contrast, the cell culture test device can be manufactured using a mold consisting of an upper mold part and a lower mold part. When the upper mold part is designed to have a downwardly tapered shape, the lower mold part is designed to have an upwardly tapered shape, and the island structures 142 and the island-connecting structures 143 are located between the upper mold part and the lower mold part, a multi-well plate having a complex shape can be manufactured by molding, as in the present disclosure (see FIG. 7). Accordingly, the cell culture test device can be manufactured by injection molding of a polymer resin. For optical observation, the cell culture test device may be made of an optically transparent polymer resin such as polystyrene, polyethylene, polypropylene, polymethacrylate or polycarbonate.

### [Features of bonding means and bonding material]

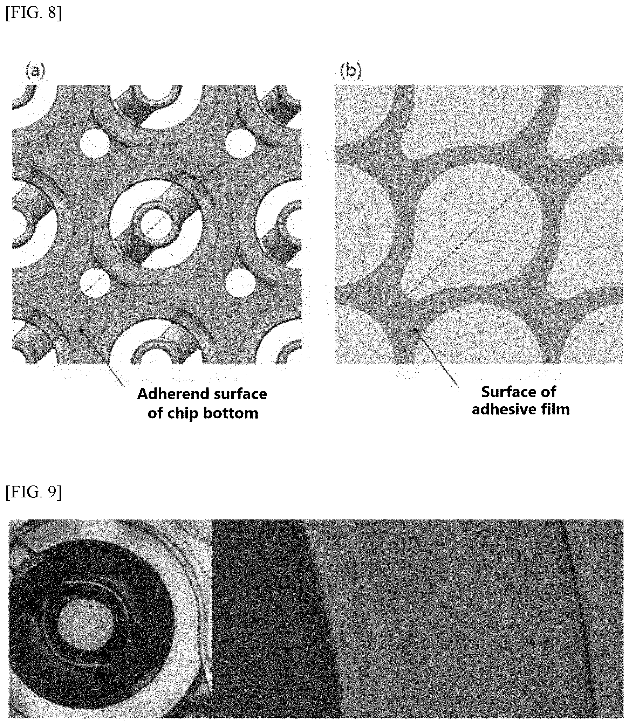

Since the lower ends of the first accommodation space 150 and the second accommodation space 120 are exposed to the outside, the first fluid and the second fluid cannot be accommodated in the first accommodation space 150 and the second accommodation space 120. Thus, bonding means 160 is bonded to the rear surface of the cell culture test device to prevent leakage of the first fluid and the second fluid and accommodate the first fluid and the second fluid in the first accommodation space 150 and the second accommodation space 120. Here, the bonding means 160 may be designed in the shape of a thin film that has the same size as the rear surface of the cell culture test device. One bonding means may be bonded to the rear surfaces of the well units. Alternatively, different bonding means may be separately bonded to the rear surfaces of the well units. In this case, each of the bonding means 160 may be adapted to the size of the corresponding well unit. It is preferable to use an optically transparent film as the bonding means because the target present in the first fluid is observed through the bottom surface of the first accommodation space. The bonding means may be an optically transparent film such as a polyethylene, polyvinyl chloride or polystyrene film. The bonding material is preferably printed only on the cell culture device or the bonding means except areas on which the bottom surfaces of the first and second accommodation spaces are placed, in order to maintain the optical transparency of the bonding means and to avoid effects caused by contact with the first fluid and the second fluid (see FIG. 8). In addition, the bonding material should not be deformed after printing and before and after bonding of the bonding means to the well units. An unreactive adhesive is recommended as the bonding material to avoid effects on the biological agent. A pressure sensitive adhesive (PSA) mainly used in adhesive tapes is preferred as the bonding material. Pressure sensitive adhesives reinforced with an ester-based adhesive based on a natural rubber, synthetic rubber, silicone rubber or acrylate may be used for the bonding material. A material having a high transmittance is preferred to avoid a reduction in the amount of light upon optical observation.

The rapid cell culture test device of the present disclosure may be designed to be openable and closable through a fastening structure or may be provided with a cover film made of silicone, polyethylene terephthalate (PET), polyethylene (PE), polypropylene (PP), polystyrene (PS), polycarbonate (PC), Teflon or polyvinyl chloride (PVC). A cruciform or X-shaped punch may be formed at a position of the cover film corresponding to the underlying first inlet portion. The punch can serve as an inlet portion through which a slight amount of the first fluid is easily introduced. Cruciform or X-shaped punches may also be formed at positions of the cover film corresponding to the underlying second inlet portion and third inlet portion. The punches can serve as inlet portions through which the fluids are easily introduced. In terms of convenience of use, it is preferable that the inlet portions through which the corresponding fluids are introduced have different shapes.

Focus marks may be provided on the underside of the second accommodation spaces to provide information on the positions of the target. The focus marks may be marked on the bottom surfaces of the first sub-wells such that the target areas of the cell culture test device can be automatically tracked. The focus marks are provided in the form of microchannels by injection molding. Since the biological agent is immobilized in the solid thin films, the use of the focus marks enables imaging of the same areas every time pictures are taken. The focus marks serve to correct the positions of the target in the three axial directions, *i.e.* x-, y-, and z-axis directions, in the automatic tracking of target areas.

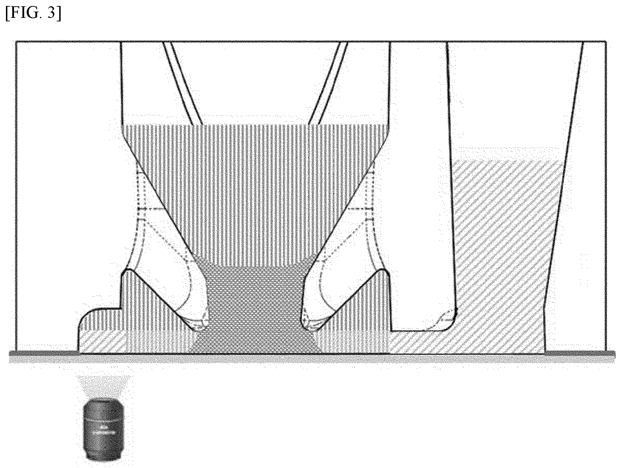

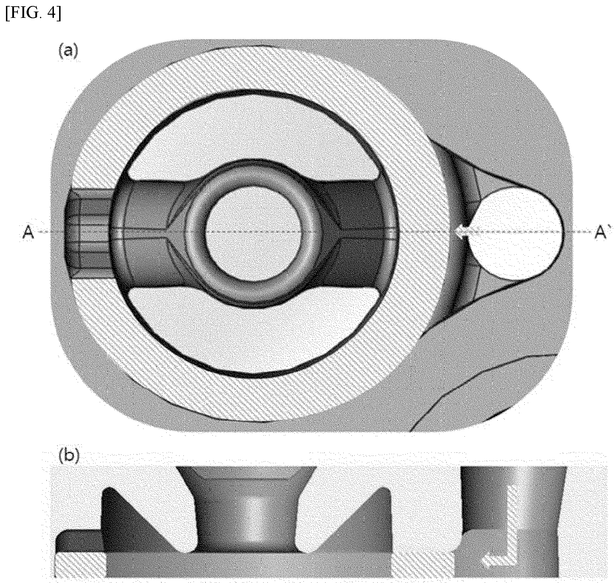

The present disclosure will be described in detail with reference to the following test method as defined in claim 13. FIG. 3 illustrates an antibiotic susceptibility test using a first fluid and a second fluid introduced into a well unit and FIG. 4 illustrates a first accommodation space to which a first fluid is supplied.

According to a further aspect of the present disclosure, there is provided a cell analysis method using a cell culture test device having an array structure of a plurality of aligned well units, each of which includes a first inlet portion through which a mixture solution of a gelling agent-containing liquid medium and a biological agent as a first fluid is introduced, a second inlet portion through which a solvent selected from water, cell culture media, dimethylformamide (DMF), and dimethyl sulfoxide (DMSO) as a second fluid is introduced, a first accommodation space in which the first fluid is accommodated, a second accommodation space in which the second fluid is accommodated, an island structure accommodated in the second accommodation space, and a third inlet portion through which an antibiotic is loaded into the island structure, the method including (a) loading the antibiotic into the third inlet portion formed above the island structure to fix the antibiotic to the inner and outer surfaces of the island structure, (b) introducing the first fluid into the first inlet portion, allowing the introduced first fluid to flow into the first accommodation space, and gelling the mixture solution to form a solid thin film, (c) introducing the second fluid into the second inlet portion, allowing the second fluid to be accommodated in the second accommodation space and come into contact with the island structure to disperse or dissolve the antibiotic, and allowing the second fluid to come into contact with the solid thin film of the first fluid in a connection portion between the first accommodation space and the second accommodation space to diffuse the antibiotic into the solid thin film, and (d) observing changes of the biological agent on a single cell basis at the interface between the solid thin film of the first fluid and the second fluid and in the solid thin film of the first fluid.

A liquid medium containing a gelling agent is mixed with a biological agent to prepare a mixture solution as a first fluid. The first fluid is prepared in a volume larger than that of the first accommodation space and is introduced into the first inlet portion. The first fluid introduced into the first inlet portion 132 flows into the first accommodation space with an appropriate dynamic contact angle under the influence of gravity and surface tension (capillary action). Generally, a flow in a flow space closed by walls is counteracted by a pressure induced by air trapped in the space. In contrast, since the first accommodation space is an open microfluidic channel connected to the second accommodation space, air is not trapped in the first accommodation space and flows freely without a pressure difference in the first accommodation space. On the other hand, a radial flow of the first fluid reaches the capillary valve connected to the second accommodation space. At this time, the angle of the wall of the capillary valve is rapidly changed so that the interface of the first fluid is inhibited from advancing as long as it does not form an appropriate dynamic contact angle with the wall after angle change. Thereafter, the first fluid is gelled and fixed only in the first accommodation space without entering the second accommodation space. As a result of the gelling, the biological agent *(e.g.,* a bacterial strain) is immobilized in the gel matrix and the first fluid forms a solid thin film that comes into contact with a second fluid, which will be described later.

Thereafter, a second fluid is introduced into the second accommodation space 120 through the second inlet portion 134 for antibiotic supply.

More specifically, the second fluid is directly introduced into the bottom of the second accommodation space 120 through an empty space surrounded by the outer wall of the island structure, the island-connecting structure 143, and the inner wall of the second accommodation space. The second fluid is introduced in an amount sufficient to submerge the island structure, so that an antibiotic can be dissolved and diffused into the second fluid. When the dissolved antibiotic is directly loaded through the third inlet portion 130, the antibiotic and the solvent may be diffused while being mixed with the second fluid. Alternatively, the antibiotic may be dried and attached to the island structure. In this case, the antibiotic is dissolved and diffused into the second fluid. Since the antibiotic-attached island structure is located in the central portion of the first accommodation space, the antibiotic is uniformly spread into the first accommodation space, and as a result, the antibiotic concentration gradient can be minimized (see FIG. 5).

The second fluid supplied to the second accommodation space reaches the capillary valve 152 located at the lower side surface of the second accommodation space and comes into contact with the first fluid. Since the first fluid is the same as or similar to the second fluid, the antibiotic is diffused into the gel matrix and changes in the growth of bacteria immobilized in the gel matrix can be observed with a microscope to determine the susceptibility of the bacteria to the antibiotic.

Next, responses of the biological agent to the antibiotic are observed. The biological agent immobilized in the solid thin film is distributed two-dimensionally, with the result that the biological agent can be observed on a single cell basis. According to the cell analysis method of the present disclosure, changes in the growth of the individual cells can be typically observed within several tens of minutes (normally 30 minutes). Accordingly, the cell analysis method of the present disclosure allows for the determination of the influence of the antibiotic on the biological agent in a more accurate and rapid manner than conventional methods. For example, the bioactivity of the antibiotic for bacterial cells can be tested within 3-4 hours. This rapid bioactivity test method is herein called single-cell morphological analysis (SCMA). The use of the cell culture test device enables observation of changes in single-cell morphology by time-lapse imaging under different antibiotic conditions.

An optical measurement system may be used for observation. The optical measurement system may include an imaging system, such as a CCD or CMOS camera. The optical measurement system may include optical units or devices necessary for focusing and light imaging, such as a lens, an illuminator, and a light guide. The optical measurement system may include an image processing system for processing and analyzing image data observed by the camera. The optical measurement system rapidly records and analyzes changes in the growth of the biological agent observed during testing to obtain test results. The area around the interface between the solid thin film of the first fluid and the second fluid is imaged. The imaging area may have a size of about 300 µm × 300 µm to 4000 µm × 4000 µm. It is preferable that the cross-sectional area of the first sub-well is at least larger in width than the imaging area.

Consequently, the use of the testing method based on the immobilization of the biological agent and the diffusion of the antibiotic can greatly reduce the amount of the drug and cells necessary for drug testing and enables rapid tracking of changes in the growth of single cells to obtain test results on the drug as rapidly as 2 hours (normally within 3-4 hours), compared to the prior art. This is the most rapid testing speed known thus far.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

Biofilms are found in areas infected with microbes or to which microbes are attached. Biofilms refer to films that constitute mucilaginous microbial complexes, which are formed by microbes surrounded by polymer matrices. The formation of biofilms can greatly affect human health. Biofilms cause pulmonary infections, otitis media, periodontitis, and other infectious diseases. The resistance of bacteria present in biofilms to antibiotics is at least 1000 times stronger than that of suspended bacteria. Flow cell systems and well-based systems have been used to investigate biofilms. However, these assay systems require a long time of several days for biofilm formation. Other difficulties associated with the use of the assay systems are that biofilms need to be stained and confocal microscopes should be used for observation. Further experiments are needed for the measurement of minimum inhibitory concentration (MIC) or minimum biofilm eradication concentration (MBEC). Such systems are very large in size and they fail to clearly show biofilm formation stages and to represent *in vivo* biofilm formation.

Thus, there is a need for efficient systems that are suitable to investigate the formation of biofilms and the reactivity of biofilms with antibiotics. In consideration of this need, the cell culture test device of the present disclosure proves to be an excellent alternative to conventional test devices.

Preferred embodiments of the present disclosure will be described in detail with reference to the accompanying drawings such that those skilled in the art can readily practice the disclosure. In describing the present disclosure, detailed explanations of a related known function or construction are omitted when it is deemed that they may unnecessarily obscure the essence of the disclosure. Certain features shown in the drawings are enlarged, reduced or simplified for ease of illustration and the drawings and the elements thereof are not necessarily in proper proportion. However, those skilled in the art will readily understand such details.

### Example 1

A thermoplastic resin (polystyrene) was injection molded into the cell culture test device illustrated in FIGS. 1 and 2, which had an array of 8 × 12 aligned well units.

Colistin as an antibiotic was loaded into the island structure through the third inlet portion of each of the well units. The antibiotic was loaded in an amount of 10 µl, dried, and fixed in the shape illustrated in (b) of FIG. 5.

Thereafter, 10 µl of a mixture of agarose and a bacterial solution (*P. aeruginosa* ATCC 27853) as a first fluid was dispensed into the first accommodation space through the first inlet portion. After the introduction of the first fluid into the first accommodation space was completed, 100 µl of a culture medium as a second fluid was introduced into the second accommodation space through the second inlet portion.

The first accommodation space was designed to have a thickness of 300 µm and a width of 1 mm. The capillary valve structure having the same thickness (300 µm) as that of the first accommodation space was formed in a contact portion between the first accommodation space and the second accommodation space. The island structure was spaced 300 µm from the bottom of the second accommodation space and was designed such that its center coincided with the center of the second accommodation space. A 100 µm thick PMMA film was used as the bonding means.

### Example 2

The procedure of Example 1 was repeated except that the thickness of the first accommodation space was changed to 500 µm and the height of the island structure from the bottom of the second accommodation space was changed to 500 µm.

### Example 3

The procedure of Example 1 was repeated except that a PET film was used as the bonding means.

### Example 4

The procedure of Example 1 was repeated except that the thickness of the first accommodation space was changed to 500 µm, the height of the island structure from the bottom of the second accommodation space was changed to 500 µm, and a PET film was used as the bonding means.

### Example 5

The procedure of Example 1 was repeated except that the thickness and width of the capillary valve structure were changed to 100 µm and 100 µm, respectively.

### Example 6

The procedure of Example 1 was repeated except that the thickness of the first accommodation space was changed to 500 µm, the height of the island structure from the bottom of the second accommodation space was changed to 500 µm, and the thickness and width of the capillary valve structure were changed to 100 µm and 100 µm, respectively.

### Example 7

The procedure of Example 1 was repeated except that the bottom of the island structure was closed.

### Example 8

The procedure of Example 1 was repeated except that the thickness of the first accommodation space was changed to 500 µm, the height of the island structure from the bottom of the second accommodation space was changed to 500 µm, and the bottom of the island structure was closed.

### Comparative Example 1

The procedure of Example 1 was repeated except that the width of the first accommodation space was changed to 0.5 mm.

### Comparative Example 2

The procedure of Example 1 was repeated except that the thickness of the first accommodation space was changed to 500 µm, the height of the island structure from the bottom of the second accommodation space was changed to 500 µm, and the width of the first accommodation space was changed to 0.5 mm.

### Comparative Example 3

The procedure of Example 1 was repeated except that the width of the first accommodation space was changed to 0.1 mm.

### Comparative Example 4

The procedure of Example 1 was repeated except that the thickness of the first accommodation space was changed to 500 µm, the height of the island structure from the bottom of the second accommodation space was changed to 500 µm, and the width of the first accommodation space was changed to 0.1 mm.

### Test Example

In each of Examples 1-8 and Comparative Examples 1-4, 10 cell culture test devices having the same structure were manufactured. A determination was made as to whether the solid thin films of the first fluid were defective. The susceptibilities to the antibiotic at four selected points of each first accommodation space were evaluated. The four points (a, b, c, and d) were at right angles to the first inlet portion. 6 h after introduction of the second fluid, *P. aeruginosa* ATCC 27853 was observed at each point to evaluate whether MIC was within the QC range for colistin approved by CLSI. The solid thin film in the first accommodation space was visually observed to determine whether the solid thin film was defective.

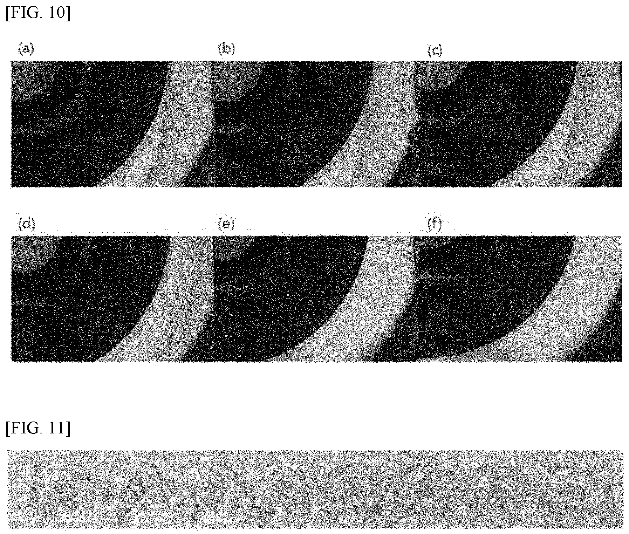

FIG. 9 shows a high magnification image of the solid thin film after 4 h without using the antibiotic colistin in Example 1 and FIG. 10 shows observation results after 6 h at various colistin concentrations: (a) 0, (b) 0.12, (c) 0.25, (d) 0.5, (e) 1, and (f) 2 mg/ml. Clear responses were observed at the concentrations. Particularly, the effect of the antibiotic was pronounced at ≥ 1 mg/ml.

**[Table 1]**

| | Number of test devices with defective solid thin films | Number of test devices having passed the QC range | | | |
|---|---|---|---|---|---|
| | | a | b | c | d |
| Example 1 | 1 | 7 | 8 | 8 | 8 |
| Example 2 | 1 | 6 | 8 | 9 | 9 |
| Example 3 | 0 | 9 | 10 | 10 | 10 |
| Example 4 | 1 | 8 | 9 | 10 | 10 |
| Example 5 | 0 | 6 | 8 | 8 | 7 |
| Example 6 | 2 | 6 | 6 | 5 | 6 |
| Example 7 | 1 | 7 | 7 | 7 | 9 |
| Example 8 | 2 | 5 | 8 | 7 | 7 |
| Comparative Example 1 | 4 | 4 | 5 | 5 | 5 |
| Comparative Example 2 | 5 | 2 | 4 | 4 | 4 |
| Comparative Example 3 | 8 | 0 | 2 | 2 | 1 |
| Comparative Example 4 | 10 | 0 | 0 | 0 | 0 |

As can be seen from the results in Table 1, none or only a few of the solid thin films in the test devices of Examples 1-8 were defective. In addition, high reliabilities of antibiotic susceptibility testing in the defect-free wells were achieved. In contrast, many defects were observed in the test devices of Comparative Examples 1-4 where the first fluid did not flow smoothly into the narrowed first accommodation spaces, failing to completely fill the first accommodation spaces, or was infiltrated into the second accommodation spaces.

The numbers of defects in the test devices of Examples 2, 4, 6, and 8 where the first accommodation spaces were relatively thick were slightly larger than those in Examples 1, 3, 5, and 7. In many of the test devices, the first fluid penetrated into the second accommodation spaces.

In the test devices of Examples 3 and 4, each of which used a more hydrophilic PET film, the first fluid did flow smoothly into the first accommodation spaces, achieving low defective rates.

In the test devices of Examples 5 and 6 where narrow capillary valves were disposed, the first fluid did flow easily into the first accommodation spaces, achieving low defective rates. However, it was difficult for the diffused antibiotic to enter the solid thin films. As a result, reduced numbers of the test devices passed the QC range.

As shown in FIG. 11, when a fluid was dispensed into the wells of Example 1 (the 1^{st}, 2^{nd}, 5^{th}, and 6^{th} from the left) and the wells of Example 2 (3^{rd}, 4^{th} 7^{th}, and 8^{th} from the left), its behaviors varied depending on the height of the island structure.

Although the particulars of the present disclosure have been described in detail, it will be obvious to those skilled in the art that such particulars are merely preferred and non-limiting embodiments.

## Claims

1. A cell culture test device comprising a plurality of well units (110),
each of which comprises a first inlet portion (132) through which a first fluid is introduced,
a first accommodation space (150) communicating with the first inlet portion (132) to accommodate a flow of the first fluid introduced through the first inlet portion (132),
a second inlet portion (134) through which a second fluid is introduced,
a second accommodation space (120) in which the introduced second fluid is accommodated,
wherein the second accommodation space (120) connects to the inner surface of the first accommodation space (150) to form an interface perpendicular to a bottom surface between the first fluid and the second fluid,
and a third inlet portion (140) through which an antibiotic has been loaded, which third inlet portion has been formed above and has been connected with an island structure (142) through an island-connecting structure (143) extending from the inner wall surface of the second accommodation space (120), wherein the antibiotic loaded from the third inlet portion (140) is fixedly accommodated on the inner and outer surfaces of the island structure (142).

2. The cell culture test device according to claim 1, wherein the center of the island structure (142) coincides with the center of the first accommodation space (150) and the island structure (142) is spaced a distance from the first accommodation space (150).

3. The cell culture test device according to claim 2, wherein the lower end of the island structure (142) is spaced 0.1 to 1 mm from the bottom surface of the second accommodation space (120) and the island structure (142) has an open bottom surface.

4. The cell culture test device according to claim 1, wherein the introduced second fluid is in contact with the inner and outer surfaces of the island structure (142), dissolves the antibiotic fixed to the inner and outer surfaces of the island structure (142), and diffuses the dissolved antibiotic into the first fluid.

5. The cell culture test device according to claim 1, wherein the thickness and width of the first accommodation space (150) are determined such that the first accommodation space (150) is filled with the first fluid introduced through the first inlet portion (132) by capillary action.

6. The cell culture test device according to claim 5, wherein the first accommodation space (150) has a width of 0.3 to 2 mm and a thickness of 100 to 500 µm.

7. The cell culture test device according to claim 1, wherein the side surface of the second accommodation space (120) is in communication with the inner surface of the first accommodation space (150) such that the first fluid and the second fluid meet each other and mass transfer to the first fluid occurs.

8. The cell culture test device according to claim 7, wherein the first accommodation space (150) is an open microfluidic structure whose at least one surface is open and the open surface of the first accommodation space (150) is connected to the side surface of the second accommodation space (120) through a capillary valve (152).

9. The cell culture test device according to claim 8, wherein the thickness and width of the capillary valve structure (152) are determined such that the introduced first fluid does not enter the second accommodation space (120) during or after flow into the first accommodation space (150).

10. The cell culture test device according to claim 9, wherein the capillary valve (152) has a thickness of 100 to 500 µm and a width of 100 µm to 2 mm.

11. The cell culture test device according to claim 1, further comprising bonding means (160) disposed on the rear surface thereof to prevent leakage of the first fluid and the second fluid.

12. The cell culture test device according to claim 11, wherein the bonding means (160) is an optically transparent film.

13. A cell analysis method using a cell culture test device having an array structure of a plurality of aligned well units (110),
each of which comprises a first inlet portion (132) through which a mixture solution of a gelling agent-containing liquid medium and a biological agent as a first fluid is introduced,
a second inlet portion (134) through which a solvent selected from water, cell culture media, dimethylformamide (DMF), and dimethyl sulfoxide (DMSO) as a second fluid is introduced,
a first accommodation space (150) communicating with the first inlet portion (132) in which the introduced first fluid is accommodated,
a second accommodation space (120) in which the introduced second fluid is accommodated,
an island structure (142) accommodated in the second accommodation space (120),
and a third inlet portion (140) through which an antibiotic is loaded into the island structure (142) and has been formed above the island structure (142) and has been connected therewith through an island-connecting structure (143) extending from the inner wall surface of the second accommodation space (120),
the method comprising (a) loading the antibiotic into the third inlet portion (140) formed above the island structure (142) to fix the antibiotic to the inner and outer surfaces of the island structure (142), (b) introducing the first fluid into the first inlet portion (132), allowing the introduced first fluid to flow into the first accommodation space (150), and gelling the mixture solution to form a solid thin film, (c) introducing the second fluid into the second inlet portion (134), allowing the second fluid to be accommodated in the second accommodation space (120) and come into contact with the island structure (142) to disperse or dissolve the antibiotic, and allowing the second fluid to come into contact with the solid thin film of the first fluid in a connection portion between the first accommodation space (150) and the second accommodation space (120) to diffuse the antibiotic into the solid thin film at their interface formed perpendicular to a bottom surface between the first fluid and the second fluid, and (d) observing changes of the biological agent on a single cell basis at the interface between the solid thin film of the first fluid and the second fluid and in the solid thin film of the first fluid.

14. The cell analysis method according to claim 13, further comprising (d) observing changes of the biological agent responding to the antibiotic on a single cell basis to determine the minimum inhibitory concentration (MIC) or minimum biofilm eradication concentration (MBEC) of the antibiotic.

## Patentansprüche

1. Zellkulturtestvorrichtung, umfassend eine Vielzahl von Well-Einheiten (110),
von denen jede umfasst: einen ersten Einlassabschnitt (132), durch den ein erstes Fluid eingeführt wird,
einen ersten Aufnahmeraum (150), der mit dem ersten Einlassabschnitt (132) in Verbindung steht, um einen Strom des durch den ersten Einlassabschnitt (132) eingeführten ersten Fluids aufzunehmen,
einen zweiten Einlassabschnitt (134), durch den ein zweites Fluid eingeführt wird,
einen zweiten Aufnahmeraum (120), in dem das eingeführte zweite Fluid aufgenommen wird,
wobei der zweite Aufnahmeraum (120) mit der Innenfläche des ersten Aufnahmeraums (150) verbunden ist, um eine Schnittfläche senkrecht zu einer Bodenfläche zwischen dem ersten Fluid und dem zweiten Fluid zu bilden,
und einen dritten Einlassabschnitt (140), durch den ein Antibiotikum geladen wurde, welcher dritte Einlassabschnitt oberhalb einer Inselstruktur (142) gebildet und mit dieser über eine Inselverbindungsstruktur (143), die sich von der Innenwandfläche des zweiten Aufnahmeraums (120) erstreckt, verbunden ist, wobei das aus dem dritten Einlassabschnitt (140) geladene Antibiotikum fest an den Innen- und Außenflächen der Inselstruktur (142) aufgenommen ist.

2. Zellkulturtestvorrichtung gemäß Anspruch 1, wobei das Zentrum der Inselstruktur (142) mit dem Zentrum des ersten Aufnahmeraums (150) zusammenfällt und die Inselstruktur (142) einen Abstand vom ersten Aufnahmeraum (150) aufweist.

3. Zellkulturtestvorrichtung gemäß Anspruch 2, wobei das untere Ende der Inselstruktur (142) einen Abstand von 0,1 bis 1 mm von der Bodenfläche des zweiten Aufnahmeraums (120) aufweist und die Inselstruktur (142) eine offene Bodenfläche aufweist.

4. Zellkulturtestvorrichtung gemäß Anspruch 1, wobei das eingeführte zweite Fluid mit den Innen- und Außenflächen der Inselstruktur (142) in Kontakt steht, das an den Innen- und Außenflächen der Inselstruktur (142) fixierte Antibiotikum auflöst und das aufgelöste Antibiotikum in das erste Fluid diffundiert.

5. Zellkulturtestvorrichtung gemäß Anspruch 1, wobei die Dicke und Breite des ersten Aufnahmeraums (150) so bestimmt sind, dass der erste Aufnahmeraum (150) mit dem durch den ersten Einlassabschnitt (132) eingeführten ersten Fluid durch Kapillarwirkung gefüllt ist.

6. Zellkulturtestvorrichtung gemäß Anspruch 5, wobei der erste Aufnahmeraum (150) eine Breite von 0,3 bis 2 mm und eine Dicke von 100 bis 500 µm aufweist.

7. Zellkulturtestvorrichtung gemäß Anspruch 1, wobei die Seitenfläche des zweiten Aufnahmeraums (120) mit der Innenfläche des ersten Aufnahmeraums (150) in Verbindung steht, so dass das erste Fluid und das zweite Fluid aufeinander treffen und ein Massetransfer zum ersten Fluid stattfindet.

8. Zellkulturtestvorrichtung gemäß Anspruch 7, wobei der erste Aufnahmeraum (150) eine offene mikrofluidische Struktur ist, deren mindestens eine Fläche offen ist, und die offene Fläche des ersten Aufnahmeraums (150) über ein Kapillarventil (152) mit der Seitenfläche des zweiten Aufnahmeraums (120) verbunden ist.

9. Zellkulturtestvorrichtung gemäß Anspruch 8, wobei die Dicke und Breite der Kapillarventilstruktur (152) so bestimmt sind, dass das eingeführte erste Fluid während oder nach dem Einströmen in den ersten Aufnahmeraum (150) nicht in den zweiten Aufnahmeraum (120) gelangt.

10. Zellkulturtestvorrichtung gemäß Anspruch 9, wobei das Kapillarventil (152) eine Dicke von 100 bis 500 µm und eine Breite von 100 µm bis 2 mm aufweist.

11. Zellkulturtestvorrichtung gemäß Anspruch 1, ferner umfassend eine an ihrer Rückseite angeordnete Verbindungsvorrichtung (160), um ein Austreten des ersten Fluids und des zweiten Fluids zu verhindern.

12. Zellkulturtestvorrichtung gemäß Anspruch 11, wobei die Verbindungsvorrichtung (160) ein optisch transparenter Film ist.

13. Zellanalyseverfahren unter Verwendung einer Zellkulturtestvorrichtung mit einer Anordnungsstruktur aus einer Vielzahl von ausgerichteten Well-Einheiten (110),
von denen jede einen ersten Einlassabschnitt (132), durch den eine Mischlösung aus einem geliermittelhaltigen flüssigen Medium und einem biologischen Agens als erstes Fluid eingeführt wird,
einen zweiten Einlassabschnitt (134), durch den ein aus Wasser, Zellkulturmedien, Dimethylformamid (DMF) und Dimethylsulfoxid (DMSO) ausgewähltes Lösungsmittel als zweites Fluid eingeführt wird,
einen ersten Aufnahmeraum (150), der mit dem ersten Einlassabschnitt (132) in Verbindung steht, in dem das eingeführte erste Fluid aufgenommen wird,
einen zweiten Aufnahmeraum (120), in dem das eingeführte zweite Fluid aufgenommen wird,
eine Inselstruktur (142), die in dem zweiten Aufnahmeraum (120) aufgenommen ist,
und einen dritten Einlassabschnitt (140), durch den ein Antibiotikum in die Inselstruktur (142) geladen wird und der über der Inselstruktur (142) gebildet und mit dieser über eine Inselverbindungsstruktur (143) verbunden ist, die sich von der Innenwandfläche des zweiten Aufnahmeraums (120) erstreckt, umfasst,
wobei das Verfahren umfasst (a) Laden des Antibiotikums in den dritten Einlassabschnitt (140), der über der Inselstruktur (142) gebildet ist, um das Antibiotikum an der Innen- und Außenfläche der Inselstruktur (142) zu fixieren, (b) Einleiten des ersten Fluids in den ersten Einlassabschnitt (132), Fließenlassen des eingeleiteten ersten Fluids in den ersten Aufnahmeraum (150) und Gelieren der Mischungslösung, um einen festen dünnen Film zu bilden, (c) Einleiten des zweiten Fluids in den zweiten Einlassabschnitt (134), Aufnehmenlassen des zweiten Fluids in dem zweiten Aufnahmeraum (120) und Inkontaktbringen mit der Inselstruktur (142), um das Antibiotikum zu dispergieren oder zu lösen, und das zweite Fluid mit dem festen dünnen Film des ersten Fluids in einem Verbindungsabschnitt zwischen dem ersten Aufnahmeraum (150) und dem zweiten Aufnahmeraum (120) in Kontakt kommen lassen, um das Antibiotikum in den festen dünnen Film an ihrer Grenzfläche, die senkrecht zu einer Bodenfläche zwischen dem ersten Fluid und dem zweiten Fluid gebildet ist, zu diffundieren, und (d) Beobachten von Veränderungen des biologischen Agens auf Einzelzellbasis an der Grenzfläche zwischen dem festen dünnen Film des ersten Fluids und dem zweiten Fluid und in dem festen dünnen Film des ersten Fluids.

14. Zellanalyseverfahren gemäß Anspruch 13, ferner umfassend (d) Beobachten von Veränderungen des biologischen Agens, das auf das Antibiotikum reagiert, auf Einzelzellbasis, um die minimale Hemmkonzentration (MIC) oder die minimale Biofilm-Eradikationskonzentration (MBEC) des Antibiotikums zu bestimmen.

## Revendications

1. Dispositif de test de culture cellulaire comprenant une pluralité d'unités de puits (110),
chacune comprenant : une première partie d'entrée (132) à travers laquelle un premier fluide est introduit,
un premier espace de réception (150) communiquant avec la première partie d'entrée (132) pour recevoir un écoulement du premier fluide introduit à travers la première partie d'entrée (132),
une seconde partie d'entrée (134) à travers laquelle un second fluide est introduit,
un second espace de réception (120) dans lequel le second fluide introduit est reçu,
où le second espace de réception (120) est relié à la surface intérieure du premier espace de réception (150) pour former une interface perpendiculaire à une surface inférieure entre le premier fluide et le second fluide,
et une troisième partie d'entrée (140) à travers laquelle un antibiotique a été chargé, cette troisième partie d'entrée ayant été formée au-dessus et ayant été connectée à une structure d'îlot (142) par l'intermédiaire d'une structure de liaison d'îlot (143) s'étendant à partir de la surface de paroi intérieure du second espace de réception (120), où l'antibiotique chargé à partir de la troisième partie d'entrée (140) est reçu de manière fixe sur les surfaces intérieure et extérieure de la structure d'îlot (142).

2. Dispositif de test de culture cellulaire selon la revendication 1, où le centre de la structure d'îlot (142) coïncide avec le centre du premier espace de réception (150), et la structure d'îlot (142) est espacée du premier espace de réception (150).

3. Dispositif de test de culture cellulaire selon la revendication 2, où l'extrémité inférieure de la structure d'îlot (142) est espacée de 0,1 à 1 mm de la surface inférieure du second espace de réception (120), et la structure d'îlot (142) a une surface inférieure ouverte.

4. Dispositif de test de culture cellulaire selon la revendication 1, où le second fluide introduit est en contact avec les surfaces intérieure et extérieure de la structure d'îlot (142), dissout l'antibiotique fixé aux surfaces intérieure et extérieure de la structure d'îlot (142) et diffuse l'antibiotique dissous dans le premier fluide.

5. Dispositif de test de culture cellulaire selon la revendication 1, où l'épaisseur et la largeur du premier espace de réception (150) sont déterminées de sorte que le premier espace de réception (150) soit rempli du premier fluide introduit à travers la première partie d'entrée (132) par capillarité.

6. Dispositif de test de culture cellulaire selon la revendication 5, où le premier espace de réception (150) a une largeur de 0,3 à 2 mm et une épaisseur de 100 à 500 µm.

7. Dispositif de test de culture cellulaire selon la revendication 1, où la surface latérale du second espace de réception (120) communique avec la surface intérieure du premier espace de réception (150) de sorte que le premier fluide et le second fluide se rencontrent et qu'un transfert de masse vers le premier fluide ait lieu.

8. Dispositif de test de culture cellulaire selon la revendication 7, où le premier espace de réception (150) est une structure microfluidique ouverte dont au moins une surface est ouverte, et la surface ouverte du premier espace de réception (150) est reliée à la surface latérale du second espace de réception (120) par l'intermédiaire d'une valve capillaire (152).

9. Dispositif de test de culture cellulaire selon la revendication 8, où l'épaisseur et la largeur de la structure de valve capillaire (152) sont déterminées de sorte que le premier fluide introduit ne passe pas dans le second espace de réception (120) pendant ou après l'entrée dans le premier espace de réception (150).

10. Dispositif de test de culture cellulaire selon la revendication 9, où la valve capillaire (152) a une épaisseur de 100 à 500 µm et une largeur de 100 µm à 2 mm.

11. Dispositif de test de culture cellulaire selon la revendication 1, comprenant en outre un dispositif de connexion (160) disposé à la surface arrière de celui-ci pour empêcher la sortie du premier fluide et du second fluide.

12. Dispositif de test de culture cellulaire selon la revendication 11, où le dispositif de connexion (160) est un film optiquement transparent.

13. Procédé d'analyse cellulaire utilisant un dispositif de test de culture cellulaire ayant une structure d'agencement d'une pluralité d'unités de puits alignées (110),
chacune comprenant : une première partie d'entrée (132) à travers laquelle une solution mixte d'un milieu liquide contenant un agent gélifiant et d'un agent biologique est introduite en tant que premier fluide,
une seconde partie d'entrée (134) à travers laquelle un solvant choisi parmi l'eau, un milieu de culture cellulaire, le diméthylformamide (DMF) et le diméthylsulfoxyde (DMSO) est introduit en tant que second fluide,
un premier espace de réception (150) communiquant avec la première partie d'entrée (132) dans lequel le premier fluide introduit est reçu,
un second espace de réception (120) dans lequel le second fluide introduit est reçu,
une structure d'îlot (142) reçue dans le second espace de réception (120),
et une troisième partie d'entrée (140) à travers laquelle un antibiotique est chargé dans la structure d'îlot (142) et formée au-dessus de la structure d'îlot (142) et reliée à celle-ci par l'intermédiaire d'une structure de liaison d'îlot (143) s'étendant à partir de la surface de paroi intérieure du second espace de réception (120),
où le procédé comprend (a) le chargement de l'antibiotique dans la troisième partie d'entrée (140) formée au-dessus de la structure d'îlot (142) pour fixer l'antibiotique aux surfaces intérieure et extérieure de la structure d'îlot (142), (b) l'introduction du premier fluide dans la première partie d'entrée (132), permettant l'écoulement du premier fluide introduit dans le premier espace de réception (150), et la gélification de la solution mixte pour former un film mince solide, (c) l'introduction du second fluide dans la seconde partie d'entrée (134), permettant la réception du second fluide dans le second espace de réception (120), et la mise en contact avec la structure d'îlot (142) pour disperser ou dissoudre l'antibiotique, et permettant la mise en contact du second fluide avec le film mince solide du premier fluide dans une partie de liaison entre le premier espace de réception (150) et le second espace de réception (120) pour diffuser l'antibiotique dans le film mince solide à son interface formée perpendiculaire à une surface inférieure entre le premier fluide et le second fluide, et (d) l'observation de changements de l'agent biologique à base de cellule unique à l'interface entre le film mince solide du premier fluide et le second fluide et dans le film mince solide du premier fluide.

14. Procédé d'analyse cellulaire selon la revendication 13, comprenant en outre (d) l'observation de changements de l'agent biologique sensible à l'antibiotique à base de cellule unique pour déterminer la concentration d'inhibition minimale (MIC) ou la concentration d'éradication de biofilm minimale (MBEC) de l'antibiotique.
